# EUROPÄISCHE PATENTSCHRIFT

(11) **EP 2 574 615 B1**
(45) Veröffentlichungstag und Bekanntmachung des Hinweises auf die Patenterteilung: **10.12.2014**
(21) Anmeldenummer: 11183622.7
(22) Anmeldetag: 30.09.2011
(51) Int. Cl.: C07H 11/04, C07F 9/09

(54) **Verfahren zur Herstellung von Zucker(thio)phosphaten**
Method for producing (thio)phosphate glucose
Procédé de fabrication de (thio)phosphates de sucre

(43) Veröffentlichungstag der Anmeldung: 03.04.2013
(73) Patentinhaber: BASF SE, 67056 Ludwigshafen (DE)
(72) Erfinder: Fleckenstein, Christoph, Dr., 63579 Freigericht (DE); Denecke, Hartmut, 67071 Ludwigshafen (DE)

(56) Entgegenhaltungen:
- WO-A1-96/05208
- WO-A1-2011/083009
- Houben-Weyl: "Organische Phosphorverbindungen", 1964, Georg Thieme Verlag, Stuttgart, XP002671338, Bd. XII/2, Seiten 371-374, * das ganze Dokument *
- H. Beyer und W. Walter: "Lehrbuch der Organischen Chemie", 1981, S. Hirzel Verlag, Stuttgart, XP002671339, Seiten 392-393, * das ganze Dokument *
- R.S. Shallenberger: "Advanced Sugar Chemistry", 1982, Ellis Horwood Limited Publishers, Chichester, XP002671340, Seiten 1-4, * Seite 1 *

## Beschreibung

Die Erfindung betrifft ein Verfahren zur Herstellung von Zucker(thio)phosphaten. Die nach diesem Verfahren hergestellten Zucker(thio)phosphate eignen sich als Flammschutzmittel in Polymeren.

Derzeit werden als Flammschutzmittel in Kunststoffen hauptsächlich polyhalogenierte Kohlenwasserstoffe, gegebenenfalls zusammen mit geeigneten Synergisten, eingesetzt. Ein typischer Vertreter dieser klassischen Flammschutzmittel ist Hexabromcyclododecan. Aufgrund von Bioakkumulation sowie Persistenz einiger polyhalogenierter Kohlenwasserstoffe ist es ein großes Bestreben der Kunststoffindustrie, halogenhaltige Flammschutzmittel durch halogenfreie zu ersetzen.

In der internationalen Anmeldung WO 2011/083009 wird Isosorbid-bis(diphenylphosphat) als halogenfreies Flammschutzmittel für Polymere vorgeschlagen. Diese Verbindung zeichnet sich durch hervorragende Flammschutzeigenschaften aus.

Gemäß WO 2011/083009 wird Isosorbid-bis(diphenylphosphat) durch Umsetzung von Isosorbid mit zwei Äquivalenten Chlordiphenylphosphat in Gegenwart der Base Triethylamin hergestellt. Als Lösungsmittel wird Toluol verwendet. Diese bislang einzige Synthese von Isosorbid-bis(diphenylphosphat) weist jedoch einige Nachteile auf, vor allem im Hinblick auf eine Durchführung im großtechnischen Maßstab.

Beispielsweise fällt im Verlauf der Reaktion Triethylammoniumhydrochlorid an, was nicht nur die gegebenenfalls mit Problemen verbundene Handhabung eines Feststoffs erforderlich macht, sondern auch zu Ausbeuteverlusten bei dessen Abtrennung führen kann. Ein weiterer Nachteil der oben beschriebenen Synthese besteht in der Notwendigkeit der Verwendung eines Lösungsmittels, wodurch die Raum-Zeit-Ausbeute der Reaktion verringert wird. Weiterhin von Nachteil sind der Einsatz des hochreaktiven Diarylchlorphosphats sowie die damit verbundene Freisetzung von Chlorid, da hierdurch die Werkstoffauswahl für Produktionsanlagen eingeschränkt wird und entsprechend resistente Materialien kostspielig sind. Ebenfalls nachteilig ist, dass die Reaktion unter Ausschluss von Feuchtigkeit durchgeführt werden muss, da Diarylchlorphosphate hydrolyselabil sind. Die Hydrolyse letzterer hätte Ausbeuteverluste sowie die Bildung unerwünschter phosphorhaltiger Nebenprodukte zur Folge.

Die Aufgabe der Erfindung liegt demnach in der Bereitstellung eines Verfahrens zur Herstellung von Zucker(thio)phosphaten, vorzugsweise des Isosorbid-bis(diphenylphosphat)-Typs, das weder die Handhabung noch die Abtrennung eines in stöchiometrischen Mengen anfallenden Feststoffs erforderlich macht und auf den Einsatz von Chlorphosphaten verzichtet. Das Verfahren soll demgemäß bezüglich Korrosionsstabilität keine besonderen Anforderungen an die Werkstoffe für Produktionsanlagen stellen, einfach durchführbar sein und die entsprechenden Produkte in hohen Ausbeuten und guten Raum-Zeit-Ausbeuten liefern.

Gelöst wird die Aufgabe durch ein Verfahren zur Herstellung eines 1,4:3,6-Dianhydrohexitol-2,5-bis(phosphats) oder-bis(thiophosphats) der Formel (II) 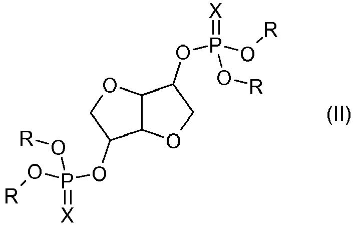 durch Umesterung von mindestens einem Phosphat oder Thiophosphat der Formel (I) 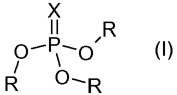 mit mindestens einem 1,4:3,6-Dianhydrohexitol der Formel (III) 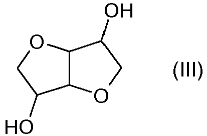 in Gegenwart von mindestens einem Katalysator aus der Gruppe der Basen und Lewis-Säuren, wobei die Substituenten X und R in den Formeln (I) und (II) folgende Bedeutung haben:
X ist gleich oder verschieden O oder S;
R ist gleich oder verschieden eine geradkettige oder verzweigte C₁-C₁₆-Alkylgruppe, eine geradkettige oder verzweigte C₂-C₁₆-Alkenylgruppe, C₃-C₁₀-Cycloalkyl, C₆-C₁₀-Aryl, Benzyl oder zwei Substituenten R bilden zusammen mit den O-Atomen, an die sie gebunden sind, und dem P-Atom einen Ring,
wobei die Substituenten R unsubstituiert oder durch einen oder mehrere Reste OH, C₁-C₄-Alkyl, CF₃, C₃-C₇-Cycloalkyl, C₁-C₄-Alkoxy, C₁-C₄-Alkenyl, C₆-C₁₀-Aryl, CN substituiert sind.

Das erfindungsgemäße Verfahren wird in Gegenwart katalytischer Mengen einer Base und/oder Lewis-Säure durchgeführt, so dass weder die Handhabung noch die Abtrennung eines in stöchiometrischen Mengen anfallenden salzartigen Feststoffs erforderlich ist. Des Weiteren kann auf die Verwendung eines Lösungsmittels verzichtet werden, wodurch die Raum-Zeit-Ausbeute der Reaktion erhöht werden kann. Das erfindungsgemäße Verfahren geht nicht von hochreaktiven Chlorphosphaten oder -thiophosphaten aus, sondern von Phosphaten der Formel P(O)(OR)₃ oder Thiophosphaten der Formel P(S)(OR)₃, und stellt damit bezüglich Chloridkorrosion keine besonderen Anforderungen an die Werkstoffe für Produktionsanlagen, sondern kann ohne Schwierigkeiten in gewöhnlichen Apparaturen durchgeführt werden. Ein weiterer Vorteil des erfindungsgemäßen Verfahrens ist, dass es nicht unter striktem Ausschluss von Feuchtigkeit durchgeführt werden muss.

WO 96/05208 offenbart ein Verfahren zur Umesterung von Phosphaten oder Thiophosphaten mit strukturell einfachen Alkoholen. Obwohl dieses Dokument eine umfassende Auflistung in Betracht kommender Nukleophile enthält, wird in den Beispielen nur die Verwendung von Phenolderivaten, primären aliphatischen Monoalkoholen und primären aliphatischen Diolen beschrieben. Ein Hinweis, dass Zucker, insbesondere 1,4:3,6-Dianhydrohexitole wie Isosorbid, als Alkohole in einer Umesterung von Phosphaten oder Thiophosphaten eingesetzt werden können, findet sich in WO 96/05208 nicht.

Es hat sich überraschenderweise gezeigt, dass Zucker mit Phosphaten der Formel P(O)(OR)₃ oder Thiophosphaten der Formel P(S)(OR)₃ zu den entsprechenden Zuckerphosphaten oder -thiophosphaten umgesetzt werden können. Zucker als hochpolare Verbindungen sind in vielen organischen Lösungsmitteln nur schwerlöslich. Au-ßerdem weisen Zucker eine hohe Dichte an funktionellen Gruppen auf, so dass bei deren Einsatz in organischen Reaktionen häufig Nebenreaktionen wie Dehydratisierungen oder Zersetzungen auftreten. Dadurch gestaltet sich der Einsatz von Zuckern in organischen Reaktionen häufig schwieriger als der von einfachen Alkoholen. Insbesondere hat sich gezeigt, dass 1,4:3,6-Dianhydrohexitole, obwohl es sich hierbei um sekundäre Diole mit zu jeweils einem Brückenatom alpha-ständigen und dadurch sterisch gehinderten Hydroxylgruppen handelt, mit Phosphaten oder Thiophosphaten zu den entsprechenden Bis(phosphaten) oder Bis(thiophosphaten) in guten Ausbeuten umgesetzt werden können.

Erfindungsgemäß umfasst der Begriff Zuckerphosphat bzw. -thiophosphat 1,4 : 3,6-Dianhydrohexitol-2,5-bis(phosphate) bzw. -bis(thiophosphate) der Formel (II)..

Im Rahmen der Erfindung umfasst der Begriff Zucker 1,4 : 3,6-Dianhydrohexitole der Formel (III).

Die Erfindung betrifft ein Verfahren zur Herstellung eines 1,4:3,6-Dianhydrohexitol-2,5-bis(phosphats) oder -bis(thiophosphats) der Formel (II) 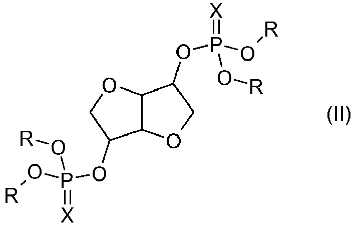 durch Umesterung von mindestens einem Phosphat oder Thiophosphat der Formel (I) mit mindestens einem 1,4:3,6-Dianhydrohexitol der Formel (III), 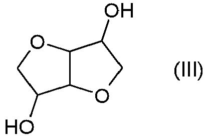 wobei die Substituenten X und R in der Formel (II) folgende Bedeutung haben:
X ist gleich oder verschieden O oder S, vorzugsweise O;
R ist gleich oder verschieden eine geradkettige oder verzweigte C₁-C₁₆-Alkylgruppe, eine geradkettige oder verzweigte C₂-C₁₆-Alkenylgruppe, C₃-C₁₀-Cycloalkyl, C₆-C₁₀-Aryl, Benzyl oder zwei Substituenten R bilden zusammen mit den O-Atomen, an die sie gebunden sind, und dem P-Atom einen Ring,
wobei die Substituenten R unsubstituiert oder durch einen oder mehrere Reste OH, C₁-C₄-Alkyl, CF₃, C₃-C₇-Cycloalkyl, C₁-C₄-Alkoxy, C₁-C₄-Alkenyl, C₆-C₁₀-Aryl, CN substituiert sind.

Geeignete 1,4:3,6-Dianhydrohexitole der Formel (III) sind 1,4:3,6-Dianhydro-D-sorbitol (D-Isosorbid, III.1), 1,4:3,6-Dianhydro-D-mannitol (D-Isomannid, III.2), 1,4:3,6-Dianhydro-L-iditol (L-Isoidid, III.3) sowie deren Enantiomere.

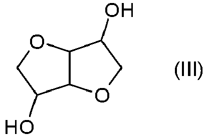

Bevorzugt sind 1,4:3,6-Dianhydro-D-sorbitol (D-Isosorbid, III.1) sowie 1,4:3,6-Dianhydro-D-mannitol (D-Isomannid, III.2).

Besonders bevorzugt ist 1,4:3,6-Dianhydro-D-sorbitol (D-Isosorbid, III.1).

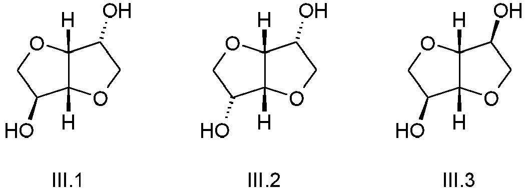

Isosorbid, Isomannid oder Isoidid können in enantiomerenreiner Form, in enantiomerenangereicherter Form oder als Racemate, in diastereomerenreiner Form, als Gemische von zwei Diastereomeren oder als Gemische von drei Diastereomeren eingesetzt werden. Bevorzugt ist die Verwendung in möglichst reiner Form.

1,4:3,6-Dianhydrohexitole sind entweder kommerziell erhältlich oder können nach in der Literatur (WO 2009/126852, WO 2007/103586, US 2004/0152907, WO 2003/089445 oder Gu, Mingyan; Yu, Dinghua; Zhang, Hongman; Sun, Peng; Huang, He; Catalysis Letters, 2009, 133, 214-220.) bekannten Methoden synthetisiert werden.

Als Phosphate oder Thiophosphate der Formel (I) eignen sich zum Beispiel Phosphate oder Thiophosphate, deren Substituenten R gleich oder verschieden eine geradkettige oder verzweigte C₁-C₁₆-Alkylgruppe, eine geradkettige oder verzweigte C₂-C₁₆-Alkenylgruppe, C₃-C₁₀-Cycloalkyl, C₆-C₁₀-Aryl, oder Benzyl sind.

Zwei Substituenten R sind gegebenenfalls miteinander verknüpft und bilden zusammen mit den O-Atomen, an die sie gebunden sind, und dem P-Atom einen Ring.

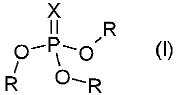

Die Substituenten R sind unsubstituiert oder durch einen oder mehrere Reste aus der Gruppe OH, C₁-C₄-Alkyl, CF₃, C₃-C₇-Cycloalkyl, C₁-C₄-Alkoxy, C₁-C₄-Alkenyl, C₆-C₁₀-Aryl, CN substituiert.

X ist gleich oder verschieden O oder S, vorzugsweise O.

Bevorzugte Substituenten R sind C₆-C₁₀-Aryl, C₁-C₄-Alkyl oder Benzyl.

Besonders bevorzugte Substituenten R sind Phenyl, Kresyl, Xylyl, Isopropylphenyl oder tert-Butylphenyl.

Weiterhin bevorzugte Substituenten R sind Methyl, Ethyl, n-Propyl, Isopropyl, Butyl, Hexyl, Cyclohexyl, Butoxyethyl oder Octyl.

In einer Ausführungsform sind zwei oder drei, vorzugsweise zwei der drei Substituenten R verschieden.

In einer bevorzugten Ausführungsform sind alle drei Substituenten R gleich.

In einer besonders bevorzugten Ausführungsform sind alle drei Substituenten R Phenyl.

Beispiele für Phosphate der Formel (I) sind Triphenylphosphat, Trikresylphosphat, Kresyldiphenylphosphat, Trixylylphosphat, Tri-(isopropylphenyl)phosphat, Tri-(tert-butylphenyl)phosphat, Diphenyloctylphosphat, Triethylphosphat, Tri-(butoxyethyl)phosphat, Tributylphosphat, Trioctylphosphat oder Diethylphenylphosphat.

Bevorzugte Phosphate sind Triphenylphosphat, Triethylphosphat oder Diethylphenylphosphat.

Ein besonders bevorzugtes Phosphat ist Triphenylphosphat.

Beispiele für Thiophosphate der Formel (I) sind Triphenylthiophosphat, Trikresylthiophosphat, Kresyldiphenylthiophosphat, Trixylylthiophosphat, Tri-(isopropylphenyl)thiophosphat, Tri-(tert-butylphenyl)thiophosphat, Diphenyloctylthiophosphat, Triethylthio-phosphat, Tributylthiophosphat, Trioctylthiophosphat oder Diethylphenylthiophosphat.

Bevorzugte Thiophosphate sind Triphenylthiophosphat, Diethylphenylthiophosphat oder Triethylthiophosphat.

Ein besonders bevorzugtes Thiophosphat ist Triphenylthiophosphat.

Es können auch Gemische von Phosphaten und/oder Thiophosphaten eingesetzt werden.

Üblicherweise werden das Phosphat oder Thiophosphat der Formel (I) und der Zucker in stöchiometrischen oder überstöchiometrischen Mengen im Verhältnis von 1:1 bis 50:1, bevorzugt 1:1 bis 25:1, besonders bevorzugt 1:1 bis 10:1, ganz besonders bevorzugt 1,5:1 bis 7,5:1 (bezogen auf eine Hydroxylgruppe des Zuckers) miteinander umgesetzt.

Das Phosphat oder Thiophosphat der Formel (I) und das 1,4:3,6-Dianhydrohexitol der Formel (III) werden im Allgemeinen in stöchiometrischen oder überstöchiometrischen Mengen im Verhältnis von 2:1 bis 100:1, bevorzugt 2:1 bis 50:1, besonders bevorzugt 2:1 bis 20:1, ganz besonders bevorzugt 3:1 bis 15:1, bezogen auf ein 1,4:3,6-Dianhydrohexitol (enthaltend 2 Hydroxylgruppen), miteinander umgesetzt.

Der Einsatz des Phosphats oder Thiophosphats und eines 1,4:3,6-Dianhydrohexitols in einem Verhältnis von <2:1 kann zur vermehrten Bildung von Verbindungen führen, die zwei oder mehrere 1,4:3,6-Dianhydrohexitoleinheiten enthalten, welche über Phosphat- oder Thiophosphatgruppen miteinander verbrückt sind. Solche Verbindungen werden im Rahmen dieser Erfindung als Dimere oder Oligomere bezeichnet. Aufgrund der Trifunktionalität des Phosphats oder Thiophosphats kann es dabei zu Vernetzungen kommen.

Ein Vorteil der Verwendung überstöchiometrischer Mengen des Phosphats oder Thiophosphats liegt darin, dass die Reaktion eines 1,4:3,6-Dianhydrohexitol-2,5-bis(phosphats) oder -(thiophosphats) der Formel (I) mit einem oder mehreren weiteren 1,4:3,6-Dianhydrohexitolen der Formel (III) oder deren Umsetzungsprodukten eingeschränkt wird.

Da viele in Betracht kommende Phosphate oder Thiophosphate unter den Bedingungen, bei denen das erfindungsgemäße Verfahren durchgeführt wird, flüssig sind, können diese auch als Lösungsmittel fungieren.

Erfindungsgemäß ist der mindestens eine Katalysator aus der Gruppe der Basen und Lewis-Säuren gewählt.

Bevorzugt ist die Wahl von ein bis drei Katalysatoren. Besonders bevorzugt ist die Wahl von ein bis zwei Katalysatoren. Ganz besonders bevorzugt ist die Wahl eines Katalysators.

In einer Ausführungsform der Erfindung ist der mindestens eine Katalysator aus der Gruppe der Basen gewählt, d.h. es wird keine Lewis-Säure eingesetzt.

Gemäß einer weiteren Ausführungsform ist der mindestens eine Katalysator aus der Gruppe der Lewis-Säuren gewählt, d.h. es wird keine Base verwendet.

In einer weiteren Ausführungsform der Erfindung werden mindestens zwei Katalysatoren eingesetzt, wobei mindestens ein Katalysator eine Base und mindestens ein Katalysator eine Lewis-Säure ist. Insbesondere bevorzugt ist dabei die Verwendung einer (1) Base in Kombination mit einer (1) Lewis-Säure.

Bevorzugt wird eine Base in Ab- oder Anwesenheit einer Lewis-Säure eingesetzt. Besonders bevorzugt ist die Verwendung einer Base in Abwesenheit einer Lewis-Säure. Als Basen eignen sich beispielsweise Alkalimetallcarbonate, z.B. Natriumcarbonat, Kaliumcarbonat, Lithiumcarbonat, Cäsiumcarbonat oder Rubidiumcarbonat, Erdalkalimetallcarbonate, z.B. Magnesiumcarbonat, Calciumcarbonat, Bariumcarbonat oder Strontiumcarbonat, Seltenerdmetallcarbonate, z.B. Lanthancarbonat oder Cercarbonat, oder Ammoniumcarbonate. Der Begriff Ammonium umfasst dabei NH₄⁺- oder Alkylammoniumionen. Ebenfalls eignen sich Alkyliminium- oder Aryliminiumcarbonate.

Weiterhin geeignete Basen sind beispielsweise Acetate wie Lithiumacetat, Natriumacetat, Kaliumacetat, Calciumacetat, Zinnacetat, Zinkacetat, Kupferacetat oder Ammoniumacetat, oder weitere Metall- oder Ammoniumcarboxylate, z.B. Propionate, Butyrate oder Benzoate. Der Begriff Ammonium umfasst dabei NH₄⁺- oder Alkylammoniumionen. Ebenfalls geeignet sind Alkyliminium- oder Aryliminiumcarboxylate, z.B. Acetate, Propionate, Butyrate oder Benzoate.

Als Basen eignen sich beispielsweise auch Alkalimetallalkoholate, z.B. Natriummethylat, Natriumethylat oder Natriumphenolat, Hydroxide, z.B. Natriumhydroxid, Kaliumhydroxid, Magnesiumhydroxid, Calciumhydroxid oder Calciumapatit, oder Oxide, z.B. Calciumoxid, Magnesiumoxid, Nickeloxid oder Strontiumoxid.

Weiterhin geeignete Basen sind zum Beispiel Metallphosphate wie Calciumphosphat oder Natriumphosphat, oder Metallhypophosphite wie Natriumhypophosphit oder Kaliumhypophosphit. Ebenfalls geeignet sind Alkyliminium- oder Aryliminiumphosphate oder -hypophosphite.

Als Basen eignen sich beispielsweise auch Alkalimetallfluoride, z.B. Natriumfluorid, Kaliumfluorid, Rubidiumfluorid oder Cäsiumfluorid, Erdalkalimetallfluoride oder Übergangsmetallfluoride.

Weiterhin geeignete Basen sind zum Beispiel organische Basen wie Ethylamin, Diethylamin, Dimethylamin, Pyridin, Pyrollidin, Piperidin, Morpholin, Chinolin, Imidazol, Chinuclidin, DABCO, DBU oder DBN.

Als Basen eignen sich beispielsweise auch geträgerte Katalysatoren, z.B. basische Ionentauscher (z.B. Lewatit VP OC 1072), basische Zeolithe, Alumina, Silica, Kreiden oder Silica-Alumina.

Bevorzugte Basen sind Carbonate oder Acetate.

Besonders bevorzugte Basen sind Kaliumcarbonat, Natriumcarbonat, Lithiumcarbonat und Lithiumacetat.

Es können auch Gemische von Basen eingesetzt werden.

Ein Vorteil des Einsatzes leichtflüchtiger Basen liegt darin, dass diese nach Beendigung der Reaktion leicht mittels Destillation aus dem Reaktionsgemisch entfernt werden können.

Beispiele für leichtflüchtige Basen umfassen Diethylamin, Triethylamin, Diisopropylamin oder Pyridin.

Weiterhin und insbesondere bevorzugt ist die Verwendung schwer- oder unlöslicher Basen, da diese sich nach Beendigung der Reaktion leicht durch Filtration teilweise oder vollständig aus dem Reaktionsgemisch entfernen lassen.

Beispiele für schwer- oder unlösliche Basen sind Natriumcarbonat, Kaliumcarbonat, Calciumcarbonat, Magnesiumcarbonat, Lithiumacetat, Natriumacetat, Calciumphosphat oder Calciumapatit

Ein Vorteil des Einsatzes leichtflüchtiger, schwerlöslicher oder unlöslicher Basen kann somit in einer schnelleren und einfacheren Aufarbeitung der Reaktionsmischung sowie Aufreinigung des Rohprodukts liegen.

Als Lewis-Säuren eignen sich zum Beispiel Magnesiumchlorid, Calciumchlorid, Aluminiumchlorid, Siliziumtetrachlorid, Siliziumtetraalkoxide, Dibutylzinnoxid oder Tetraalkyltitanate wie Tetrabutyltitanat, Tetramethyltitanat oder Tetraisopropyltitanat.

Bevorzugte Lewis-Säuren sind Magnesiumchlorid, Calciumchlorid und Tetraisopropyltitanat.

Der Katalysator wird in einer katalytischen Menge verwendet, was im Rahmen dieser Erfindung bedeutet, dass der Katalysator in einer Menge im Bereich von 0,02 bis 10 mol%, vorzugsweise 0,05 bis 5 mol%, besonders bevorzugt 0,2 bis 3 mol% und ganz besonders bevorzugt 0,5 bis 2 mol%, bezogen auf die Menge an eingesetztem Zucker, vorzugsweise 1,4:3,6-Dianhydrohexitol, verwendet wird.

Im Allgemeinen wird das erfindungsgemäße Verfahren folgendermaßen durchgeführt:
Ein oder mehrere Phosphate oder Thiophosphate der Formel (I), ein oder mehrere Zucker, vorzugsweise 1,4:3,6-Dianhydrohexitole der Formel (III) sowie ein oder mehrere Basen und/oder Lewis-Säuren werden im Allgemeinen bei Temperaturen im Bereich von 60 bis 200 °C, vorzugsweise 90 bis 170 °C, besonders bevorzugt 110 bis 150 °C, über einen Zeitraum von 0,1 bis 10 h, vorzugsweise 0,5 bis 5 h, besonders bevorzugt 0,5 bis 4 h miteinander umgesetzt.

In einer besonders bevorzugten Ausführungsform der Erfindung fungiert das eingesetzte Phosphat oder Thiophosphat als Lösungsmittel.

Das erfindungsgemäße Verfahren kann in Anwesenheit eines Verdünnungsmittels durchgeführt werden. Als Verdünnungsmittel eignen sich Kohlenwasserstoffe, z.B. Benzol, Toluol, Xylol, Mesitylen oder Chlorbenzol; Ether, z.B. Dibutylether oder Dioxan; dipolar aprotische Lösungsmittel, z.B. DMF, DMSO oder Acetonitril. Bevorzugte Lösungsmittel sind Xylol, Mesitylen, Chlorbenzol oder Dibutylether. Es können auch Gemische dieser Lösungsmittel eingesetzt werden. Gemäß der Erfindung handelt es sich bei einem Verdünnungsmittel um ein Mittel, das den eingesetzten Zucker, vorzugsweise das eingesetzte 1,4:3,6-Dianhydrohexitol, und das eingesetze Phosphat oder Thiophosphat verdünnt.

In einer bevorzugten Ausführungsform wird das Verfahren in Abwesenheit eines Verdünnungsmittels durchgeführt.

Die Durchführung des erfindungsgemäßen Verfahrens erfolgt üblicherweise bei Drücken im Bereich von 0 bis 1013 mbar, bevorzugt 0 bis 200 mbar, besonders bevorzugt 0 bis 20 mbar.

In einer Ausführungsform wird das Verfahren bei Atmosphärendruck durchgeführt. Unter Atmosphärendruck versteht man im Rahmen der Erfindung einen Druck im Bereich von 1003 bis 1023 mbar, vorzugsweise 1013 mbar.

In einer bevorzugten Ausführungsform wird das Verfahren unter Vakuum durchgeführt. Dadurch werden flüchtige Kondensationsnebenprodukte, z.B. Phenol(derivate) oder andere Alkohole, entfernt und das Reaktionsgleichgewicht zu Gunsten der Produkte verschoben. Im Allgemeinen wird ein vollständiger Umsatz des eingesetzten Zuckers, vorzugsweise 1,4:3,6-Dianhydrohexitols erreicht. Unter Vakuum versteht man im Rahmen der Erfindung einen Druck im Bereich von 0 bis 10 mbar, vorzugsweise 0 bis 5 mbar, insbesondere bevorzugt 0 bis 1 mbar.

Erfolgt die Durchführung des Verfahrens bei vermindertem Druck, so wird es vorzugsweise in Abwesenheit eines Verdünnungsmittels durchgeführt.

In einer bevorzugten Ausführungsform wird das Verfahren bei Drücken im Bereich von 0 bis 20 mbar und in Abwesenheit eines Verdünnungsmittels durchgeführt.

Die Aufarbeitung der Reaktionsgemische erfolgt beispielsweise durch Filtration (Abtrennung des Katalysators) und/oder Destillation (Entfernung von Kondensatnebenprodukten, z.B. Phenol(derivate) oder andere Alkohole, oder von überschüssigem Phosphat oder Thiophosphat, z.B. Triphenylphosphat). Die Produkte fallen zum Teil in Form zäher Öle an, die unter vermindertem Druck und bei mäßig erhöhter Temperatur von flüchtigen Anteilen befreit oder gereinigt werden. Sofern die Produkte als Feststoffe erhalten werden, kann die Reinigung auch durch Umkristallisieren oder Digerieren erfolgen.

In einer besonderen Ausführungsform werden im Reaktionsaustrag verbleibende phenolische Kondensate extraktiv, z.B. durch wässrig-alkalische Extraktion, abgereichert. Unter einem phenolischen Kondensat versteht man im Sinne der Erfindung Phenol und/oder ein oder mehrere Phenolderivate, die als Nebenprodukt(e) bei der Umesterung gemäß einer bevorzugten Ausführungsform anfallen.

Im Allgemeinen liegen die Ausbeuten in einem Bereich von 70 bis >99%, vorzugsweise 85 bis >99%.

Die nach dem erfindungsgemäßen Verfahren hergestellten Bis(phosphate) eignen sich beispielsweise als Flammschutzmittel in Polymeren. Bevorzugt ist die Verwendung in Schaumstoffen, insbesondere in auf Polystyrol basierenden Schaumstoffen.

Das erfindungsgemäß hergestellte Flammschutzmittel wird entweder allein oder in Mischung mit weiteren flammhemmenden Substanzen und/oder mit Synergisten für die Herstellung flammhemmend ausgerüsteter (bzw. flammgeschützter) Polymere, insbesondere thermoplastischer Polymere, verwendet. Hierfür werden die Flammschutzmittel vorzugsweise physikalisch mit dem entsprechenden Polymer in der Schmelze vermischt und dann entweder als Polymermischung mit Phosphorgehalten zwischen 0,05 Gew.-Teilen und 5 Gew.-Teilen (bezogen auf das Polymer) zunächst fertig konfektioniert und dann in einem zweiten Verfahrensschritt zusammen mit demselben oder mit einem anderen Polymer weiterverarbeitet. Alternativ ist im Falle von Styrolpolymeren auch der Zusatz des Flammschutzmittels vor, während und/oder nach der Herstellung durch Suspensionspolymerisation bevorzugt.

Als Polymer können beispielsweise geschäumte oder ungeschäumte Styrolpolymere, einschließlich ABS, ASA, SAN, AMSAN, SB und HIPS Polymere, Polyimide, Polysulfone, Polyolefine wie Polyethylen und Polypropylen, Polyacrylate, Polyetheretherketone, Polyurethane, Polycarbonate, Polyphenylenoxide, ungesättigte Polyesterharze, Phenolharze, Polyamide, Polyethersulfone, Polyetherketone und Polyethersulfide, jeweils einzeln oder in Mischung als Polymerblends eingesetzt werden.

Bevorzugt sind thermoplastische Polymere wie geschäumte oder ungeschäumte Styrolhomopolymere und -copolymere jeweils einzeln oder in Mischung als Polymerblends.

Erfindungsgemäß umfasst der Begriff Styrolpolymer Polymere auf Basis von Styrol, alpha-Methylstyrol oder Mischungen von Styrol- und alpha-Methylstyrol; analog gilt dies für den Styrolanteil in SAN, AMSAN, ABS, ASA, MBS und MABS (siehe unten). Erfindungsgemäße Styrolpolymere basieren auf mindestens 50 Gew.-Teilen Styrol und/oder alpha-Methylstyrol Monomeren.

Bevorzugt werden als Styrolpolymere glasklares Polystyrol (GPPS), Schlagzähpolystyrol (HIPS), anionisch polymerisiertes Polystyrol oder Schlagzähpolystyrol (A-IPS), Styrol-alpha-Methylstyrol-copolymere, Acrylnitril-Butadien-Styrolpolymerisate (ABS), Styrol-Acrylnitril-Copolymere (SAN), Acrylnitril-alpha-Methylstyrol-Copolymere (AMSAN), Acrylnitril-Styrol-Acrylester (ASA), Methylacrylat-Butadien-Styrol (MBS), Methylmethacrylat-Acrylnitril-Butadien-Styrol (MABS)-polymerisate oder Mischungen davon oder mit Polyphenylenether (PPE) eingesetzt.

Die genannten Styrolpolymere können zur Verbesserung der mechanischen Eigenschaften oder der Temperaturbeständigkeit gegebenenfalls unter Verwendung von Verträglichkeitsvermittlern mit thermoplastischen Polymeren, wie Polyamiden (PA), Polyolefinen wie Polypropylen (PP) oder Polyethylen (PE), Polyacrylaten wie Polymethylmethacrylat (PMMA), Polycarbonat (PC), Polyestern wie Polyethylenterephthalat (PET) oder Polybutylenterephthalat (PBT), Polyethersulfonen (PES), Polyetherketonen oder Polyethersulfiden (PES) oder Mischungen davon in Anteilen von insgesamt bis maximal 30 Gew.-Teilen, bevorzugt im Bereich von 1 bis 10 Gew.-Teilen, bezogen auf 100 Gew.-Teile der Polymerschmelze, enthalten. Des Weiteren sind Mischungen in den genannten Mengenbereichen auch mit zum Beispiel hydrophob modifizierten oder funktionalisierten Polymeren oder Oligomeren, Kautschuken wie Polyacrylaten oder Polydienen, zum Beispiel Styrol-Butadien-Blockcopolymeren oder biologisch abbaubaren aliphatischen oder aliphatisch/aromatischen Copolyestern möglich.

Bevorzugt sind flammgeschützte Polymerschaumstoffe, insbesondere auf Basis von Styrolpolymeren, vorzugsweise EPS und XPS.

Die expandierbaren Styrolpolymere (EPS) können entweder im Extrusionsverfahren oder mittels Suspensionspolymerisation in wässriger Suspension in Gegenwart des erfindungsgemäßen Flammschutzmittels und eines organischen Treibmittels hergestellt werden.

Die flammgeschützten Polymerschaumstoffe weisen bevorzugt eine Dichte im Bereich von 5 bis 200 kg/m³, besonders bevorzugt im Bereich von 10 bis 50 kg/m³, auf und sind bevorzugt zu mehr als 80%, besonders bevorzugt zu 90 bis 100% geschlossenzellig.

Für die Verwendung in EPS sind vollständig phosphatisierte oder thiophosphatisierte Hydroxylgruppen der nach dem erfindungsgemäßen Verfahren hergestellten Zuckeroligo(phosphate) oder -oligo(thiophosphate) bevorzugt.

Die Erfindung wird durch die nachfolgenden Beispiele näher erläutert.

### Beispiele

### A. Synthesebeispiel

Exemplarisch für eine Synthesevorschrift sei an dieser Stelle eine Vorschrift für die Synthese von Isosorbid-bis(diphenylphosphat) durch Umesterung von Triphenylphosphat mit Isosorbid angegeben.

### Synthese von Isosorbid-bis(diphenylphosphat):

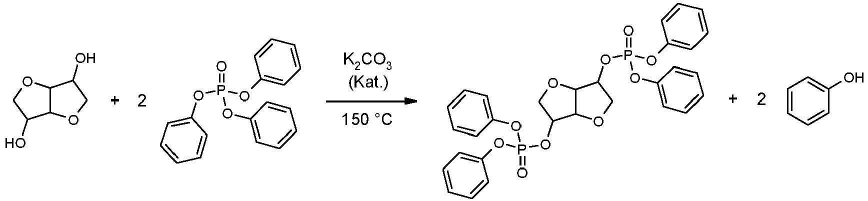

### Apparatur:

2L-Rührapparatur, Teflonblattrührer, Thermometer, Ölbad, mit Thermostat beheizte (45 °C) Claisenbrücke.

### Ansatz:

| | | |
|---|---|---|
| 119,8g | 0,82 mol | Isosorbid |
| 535,1g | 1,64 mol | Triphenylphosphat |
| 1,13g | 8,2 mmol | Kaliumcarbonat |

In einer 2L-Standardrührapparatur wurden Isosorbid (119,8 g, 0,82 mol), Triphenylphosphat (535,1 g, 1,64 mol) und Kaliumcarbonat (1,13 g, 8,2 mmol) vorgelegt. Der Ansatz wurde 2 h bei 150 °C im Vakuum (1 mbar) gerührt. Das Reaktionsgemisch lag hierbei als niedrigviskose, leicht rührbare Schmelze vor. Während dieser Zeit wurde Phenol (128 g, 83% d. Th.) als Destillat aufgefangen.

Der Reaktionsrückstand wurde bei 80 °C über eine Glasfritte (Por. 3) filtriert, um verbleibende Feststoffpartikel (Katalysator) abzutrennen. Anschließend wurde das Filtrat einer Molekulardestillation unterzogen, um nicht umgesetzes Triphenylphosphat sowie Reste an Phenol zu entfernen.

Das Produkt (Sumpf der Molekulardestillation) wurde als leicht gelbliches Öl (441 g, 88% d. Th.) isoliert. Die Produktzusammensetzung wurde mittels ³¹P-NMR wie folgt bestimmt:

| **Produktzusammensetzung (%)*** | | | |
|---|---|---|---|
| Triphenylphosphat (-17 ppm) | Isosorbid-bis(diphenylphosphat) (-12 bis -13 ppm) | Dimere** (-8 bis -7 ppm) | Oligomere*** (-4 bis -2 ppm) |
| 1 | 76 | 20 | 3 |

| | | | |
|---|---|---|---|
| * bestimmt mittels ³¹P-NMR unter Verwendung von Triphenylphosphin als Standard, Probe gelöst in CDCl₃. ** Der Begriff Dimer bezeichnet zum Beispiel eine Verbindung der folgenden Struktur: 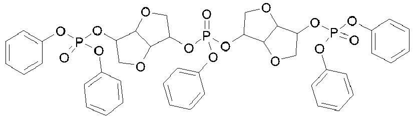 *** Der Begriff Oligomer bezeichnet beispielsweise Verbindungen der folgenden Struktur mit n>2 und umfasst neben linearen auch verzweigte Strukturen: 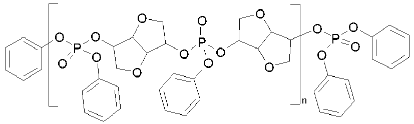 | | | |

### B. Anwendungsbeispiele

### Beschreibung der Versuche:

Die Ermittlung des Brandverhaltens der Schaumstoffplatten erfolgte, wenn nicht anders angegeben, bei einer Schaumstoffdichte von 15 kg/m³ nach DIN 4102 (Brandtest B2).

Als Vergleich wurden Hexabromcyclododecan (im Folgenden als HBCD bezeichnet) sowie Triphenylphosphat eingesetzt.

### Expandierbare Styrolpolymerisate (Extrusionsprozess):

7 Gew.-Teile n-Pentan wurden in eine Polystyrolschmelze aus PS 148H (Mw = 240 000 g/mol, Mn = 87 000 g/mol, bestimmt mittels GPC, RI-Detektor, Polystyrol (PS) als Standard) der BASF SE mit einer Viskositätszahl VZ von 83 ml/g eingemischt. Nach Abkühlen der treibmittelhaltigen Schmelze von ursprünglich 260 °C auf eine Temperatur von 190 °C wurde eine Polystyrolschmelze, welche die in der Tabelle genannten Flammschutzmittel und optional Schwefel (Synergist) enthielt, über einen Seitenstromextruder in den Hauptstrom eingemischt.

Die angegebenen Mengen in Gew.-Teilen beziehen sich auf die gesamte Polystyrolmenge (100 Gew.-Teile).

Das Gemisch aus Polystyrolschmelze, Treibmittel und Flammschutzmittel wurde mit 60 kg/h durch eine Düsenplatte mit 32 Bohrungen (Durchmesser der Düsen 0,75 mm) gefördert. Mit Hilfe einer druckbeaufschlagten Unterwassergranulierung wurden kompakte Granulate mit enger Größenverteilung hergestellt.

Das Molekulargewicht der Granulate betrug 220 000 g/mol (Mw) bzw. 80 000 g/mol (Mn) (bestimmt mittels GPC, RI-Detektor, PS als Standard). Durch Einwirkung von strömendem Wasserdampf wurden die Granulate vorgeschäumt und nach 12-stündiger Lagerung durch weitere Behandlung mit Wasserdampf in einer geschlossenen Form zu Schaumstoffblöcken einer Dichte von 15 kg/m³ verschweißt. Die Ermittlung des Brandverhaltens der Schaumstoffplatten erfolgte nach 72-stündiger Lagerung bei einer Schaumstoffdichte von 15 kg/m³ nach DIN 4102.

Die Ergebnisse sind in Tabelle 1 (Beispiele 1-3) zusammengestellt.

**Tabelle 1: Brandverhalten von Polystyrol, das nach dem erfindungsgemäßen Verfahren hergestelltes Isosorbid-bis(diphenylphosphat) als Flammschutzmittel enthält, und Vergleichsbeispiele**

| **Beispiel** | **Flammschutzmittel** (Gew.-Teile bezogen auf 100 Gew.-Teile Polystyrol) | **Synergist** (Gew.-Teile bezogen auf 100 Gew.-Teile Polystyrol) | **Brandtest** (B2 nach DIN 4102) / **Verlöschzeit** (s) |
|---|---|---|---|
| **VB1** | - | - | nicht bestanden / brennt ab |
| **VB2** | HBCD (4,0) | - | bestanden / 6,6 |
| **VB3** | TPP^{a} (5,0) | Schwefel (2,5) | bestanden / 9,5 |
| **VB4** | TPP^{a} (2,5) | Schwefel (2,5) | nicht bestanden / brennt ab |
| **VB5** | Isosorbid-bis(diphenyl-phosphat)^{b} (5,0) | Schwefel (2,5) | bestanden / 6,3 |
| **VB6** | Isosorbid-bis(diphenyl-phosphat)^{b} (2,5) | Poly-(tert-butyl-phenoldisulfid)^{c} (2,5) | bestanden / 5.8 |
| **1** | Isosorbid-bis(diphenyl-phosphat)^{d} (5,0) | Schwefel (2,5) | bestanden / 6,7 |
| **2** | Isosorbid-bis(diphenyl-phosphat)^{d} (2,5) | Schwefel (2,5) | bestanden / 8,1 |
| **3** | Isosorbid-bis(diphenyl-phosphat)^{d} (1,0) | Schwefel (2,5) | bestanden /10,4 |
| **4** | Isosorbid-bis(diphenyl-phosphat)^{d} (2,5) | Poly-(tert-butyl-phenoldisulfid)^{c} (2.5) | bestanden / 6,9 |

| | | | |
|---|---|---|---|
| ^{a} Triphenylphosphat, kommerziell erhältlich als Disflamoll^{®} TP von der Firma Lanxess; ^{b} hergestellt nach der in WO 2011/083009 vorgeschlagenen Methode durch Umsetzung von Chlordiphenylphosphat mit Isosorbid in Gegenwart von Triethylamin (stöchiometrisch); ^{c} kommerziell erhältlich als Vultac^{®} TB7 von der Firma Arkema; ^{d} hergestellt nach dem erfindungsgemäßen Verfahren durch Umesterung von TPP mit Isosorbid in Gegenwart von Kaliumcarbonat (katalytisch). | | | |

### Extrudierte Polystyrol-Schaumstoffplatten:

100 Gew.-Teile Polystyrol 158K (Mw = 261 000 g/mol, Mn = 77 000 g/mol bestimmt mittels GPC, RI-Detektor, PS als Standard) der BASF SE mit einer Viskositätszahl von 98 ml/g, 0,1 Teile Talkum als Keimbildner zur Regelung der Zellgröße und die in der Tabelle angegebenen Teile an Flammschutzmitteln sowie gegebenenfalls Schwefel (Synergist) wurden in einem Extruder mit einem inneren Schneckendurchmesser von 120 mm kontinuierlich zugeführt. Durch eine in den Extruder angebrachte Einlassöffnung wurde gleichzeitig ein Treibmittelgemisch aus 3,25 Gew.-Teilen Ethanol und 3,5 Gew.-Teilen CO₂ kontinuierlich eingedrückt. Das in dem Extruder bei 180 °C gleichmä-ßig geknetete Gel wurde durch eine Beruhigungszone geführt und nach einer Verweilzeit von 15 Minuten mit einer Austrittstemperatur von 105 °C durch eine 300 mm breite und 1,5 mm weite Düse in die Atmosphäre extrudiert. Der Schaum wurde durch einen mit dem Extruder verbundenen Formkanal geführt, wobei eine geschäumte Plattenbahn mit einem Querschnitt 650 mm x 50 mm und einer Dichte von 40 g/l entstand. Das Molekulargewicht des Polystyrols betrug 240 000 g/mol (Mw) bzw. 70 000 g/mol (Mn) (bestimmt mittels GPC, RI-Detektor, PS als Standard). Das Produkt wurde in Platten geschnitten. Geprüft wurde das Brandverhalten der Proben mit Dicken von 35 mm nach einer Ablagerungszeit von 30 Tagen nach DIN 4102.

Die Ergebnisse sind in Tabelle 2 (Beispiele 4-6) zusammengefasst.

**Tabelle 2: Brandverhalten von Polystyrol, das nach dem erfindungsgemäßen Verfahren hergestelltes Isosorbid-bis(diphenylphosphat) als Flammschutzmittel enthält, und Vergleichsbeispiele**

| **Beispiel** | **Flammschutzmittel** (Gew.-Teile bezogen auf 100 Gew.-Teile Polystyrol) | **Synergist** (Gew.-Teile bezogen auf 100 Gew.-Teile Polystyrol) | **Brandtest** (B2 nach DIN 4102) / **Verlöschzeit** (s) |
|---|---|---|---|
| **VB7** | - | - | nicht bestanden / brennt ab |
| **VB8** | HBCD (4,0) | - | bestanden /7,2 s |
| **VB9** | TPP^{a} (5,0) | Schwefel (2,5) | bestanden /10,7 s |
| **VB10** | TPP^{a} (2,5) | Schwefel (2,5) | nicht bestanden / brennt ab |
| **VB11** | Isosorbid-bis(diphenyl-phosphat^{b} (5,0) | Schwefel (2,5) | bestanden / 6,0 |
| **VB12** | Isosorbid-bis(diphenyl-phosphat^{b} (2,5) | Poly-(tert-butyl-phenoldisulfid)^{c} (2,5) | bestanden / 7,1 |
| **5** | Isosorbid-bis(diphenyl-phosphat)^{d} (5,0) | Schwefel (2,5) | bestanden / 6,5 |
| **6** | Isosorbid-bis(diphenyl-phosphat)^{d} (2,5) | Schwefel (2,5) | bestanden / 7,1 |
| **7** | Isosorbid-bis(diphenyl-phosphat)^{d} (1,0) | Schwefel (2,5) | bestanden / 9,9 |
| **8** | Isosorbid-bis(diphenyl-phosphat)^{d} (2,5) | Poly-(tert-butylphenoldisulfid)^{c} (2,5) | bestanden / 6,3 |

| | | | |
|---|---|---|---|
| ^{a} Triphenylphosphat, kommerziell erhältlich als Disflamoll^{®} TP; ^{b} hergestellt nach der in WO 2011/083009 vorgeschlagenen Methode durch Umsetzung von Chlordiphenylphosphat mit Isosorbid in Gegenwart von Triethylamin (stöchiometrisch); ^{c} kommerziell erhältlich als Vultac^{®} TB7 von der Firma Arkema; ^{d} hergestellt nach dem erfindungsgemäßen Verfahren durch Umesterung von TPP mit Isosorbid in Gegenwart von Kaliumcarbonat (katalytisch). | | | |

Polystyrol-Schaumstoffplatten, die nach dem erfindungsgemäßen Verfahren hergestelltes Isosorbid-bis(diphenylphosphat) als Flammschutzmittel enthalten, zeigen ein mit entsprechenden Schaumstoffplatten, die nach der in WO 2011/083009 vorgeschlagenen Methode hergestelltes Isosorbid-bis(diphenylphosphat) enthalten, vergleichbares Brandverhalten. Das erfindungsgemäße Verfahren zur Herstellung der Flammschutzmittel bietet jedoch signifikante Vorteile gegenüber der in WO 2011/083009 vorgeschlagenen Methode. Ersteres verzichtet auf den Einsatz von kostspieligen Chlorphosphaten oder von stöchiometrischen Mengen einer Base und zeichnet sich somit durch einen geringeren Materialaufwand und niedrigere Rohstoffkosten aus. Bezüglich Chloridkorrosion stellt es keine besonderen Anforderungen an die Produktionsanlagen, was mit einem geringeren Anlageninvest verbunden ist. Weitere Vorteile des erfindungsgemäßen Verfahrens sind dessen einfachere Durch- und Reaktionsführung, eine geringere Anzahl an Prozessschritten sowie höhere Raum-Zeit-Ausbeuten.

## Patentansprüche

1. Verfahren zur Herstellung eines 1,4:3,6-Dianhydrohexitol-2,5-bis(phosphats) oder -bis(thiophosphats) der Formel (II) 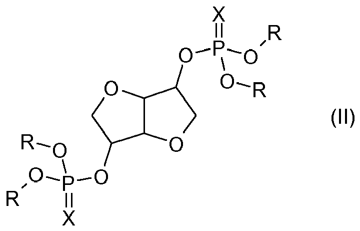 durch Umesterung von mindestens einem Phosphat oder Thiophosphat der Formel (I) 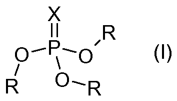 mit mindestens einem 1,4:3,6-Dianhydrohexitol der Formel (III) 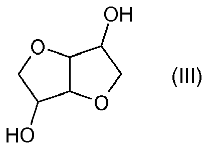 in Gegenwart von mindestens einem Katalysator aus der Gruppe der Basen und Lewis-Säuren, wobei die Substituenten X und R in den Formeln (I) und (II) folgende Bedeutung haben:
X ist gleich oder verschieden O oder S;
R ist gleich oder verschieden eine geradkettige oder verzweigte C₁-C₁₆-Alkylgruppe, eine geradkettige oder verzweigte C₂-C₁₆-Alkenylgruppe, C₃-C₁₀-Cycloalkyl, C₆-C₁₀-Aryl, Benzyl oder zwei Substituenten R bilden zusammen mit den O-Atomen, an die sie gebunden sind, und dem P-Atom einen Ring,
wobei die Substituenten R unsubstituiert oder durch einen oder mehrere Reste OH, C₁-C₄-Alkyl, CF₃, C₃-C₇-Cycloalkyl, C₁-C₄-Alkoxy, C₁-C₄-Alkenyl, C₆-C₁₀-Aryl, CN substituiert sind.

2. Verfahren gemäß Anspruch 1, **dadurch gekennzeichnet, dass** alle Substituenten X gleich O sind.

3. Verfahren gemäß Anspruch 1, **dadurch gekennzeichnet, dass** alle Substituenten X gleich S sind.

4. Verfahren gemäß einem der Ansprüche 1 bis 3, **dadurch gekennzeichnet, dass** R gleich oder verschieden C₆-C₁₀-Aryl ist.

5. Verfahren gemäß einem der Ansprüche 1 bis 4, **dadurch gekennzeichnet, dass** alle Substituenten R gleich sind.

6. Verfahren gemäß einem der Ansprüche 1 bis 5, **dadurch gekennzeichnet, dass** alle Substituenten R Phenyl sind.

7. Verfahren gemäß einem der Ansprüche 1 bis 6, **dadurch gekennzeichnet, dass** der mindestens eine Katalysator aus der Gruppe der Basen gewählt ist.

8. Verfahren gemäß einem der Ansprüche 1 bis 7, **dadurch gekennzeichnet, dass** die Base Kaliumcarbonat, Natriumcarbonat, Lithiumcarbonat oder Lithiumacetat ist.

9. Verfahren gemäß einem der Ansprüche 1 bis 6, **dadurch gekennzeichnet, dass** der mindestens eine Katalysator aus der Gruppe der Lewis-Säuren gewählt ist.

10. Verfahren gemäß einem der Ansprüche 1 bis 6, 8 oder 9, **dadurch gekennzeichnet, dass** die Lewis-Säure Magnesiumchlorid, Calciumchlorid oder Tetraisopropyltitanat ist.

11. Verfahren gemäß einem der Ansprüche 1 bis 10, **dadurch gekennzeichnet, dass** das Phosphat oder Thiophosphat der Formel (I) und das 1,4:3,6-Dianhydrohexitol der Formel (III) in stöchiometrischen oder überstöchiometrischen Mengen im Verhältnis von 2:1 bis 20:1, bezogen auf ein 1,4:3,6-Dianhydrohexitol, miteinander umgesetzt werden.

12. Verfahren gemäß einem der Ansprüche 1 bis 11, **dadurch gekennzeichnet, dass** es bei Temperaturen im Bereich von 90 bis 170 °C durchgeführt wird.

13. Verfahren gemäß einem der Ansprüche 1 bis 12, **dadurch gekennzeichnet, dass** es in Abwesenheit eines Verdünnungsmittels durchgeführt wird.

14. Verfahren gemäß Anspruch 13, **dadurch gekennzeichnet, dass** es bei Drücken im Bereich von 0 bis 20 mbar durchgeführt wird.

## Claims

1. Process for producing a 1,4:3,6-dianhydrohexitol 2,5-bis(phosphate) or 1,4:3,6-dianhydrohexitol 2,5-bis(thiophosphate) of the formula (II) 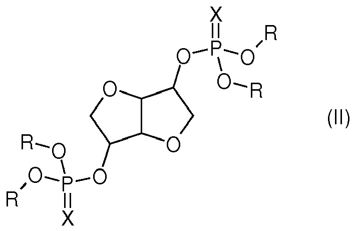 via transesterification of at least one phosphate or thiophosphate of the formula (I) 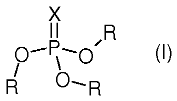 with at least one 1,4:3,6-dianhydrohexitol of the formula (III), 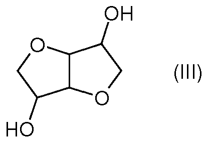 in the presence of at least one catalyst from the group of the bases and Lewis acids,
where the definitions of the substituents X and R in the formulae (I) and (II) are as follows:
X, being identical or different, is O or S;
R, being identical or different, is a straight-chain or branched C₁-C₁₆-alkyl group, a straight-chain or branched C₂-C₁₆-alkenyl group, C₃-C₁₀-cycloalkyl, C₆-C₁₀-aryl, or benzyl, or two substituents R together with the O atoms bonded thereto and with the P atom form a ring,
where the substituents R are unsubstituted or have substitution by one or more OH, C₁-C₄-alkyl, CF₃, C₃-C₇-cycloalkyl, C₁-C₄-alkoxy, C₁-C₄-alkenyl, C₆-C₁₀-aryl, or CN moieties.

2. The process according to claim 1, wherein all of the substituents X are O.

3. The process according to claim 1, wherein all of the substituents X are S.

4. The process according to any of claims 1 to 3, wherein R, being identical or different, is C₆-C₁₀-aryl.

5. The process according to any of claims 1 to 4, wherein all of the substituents R are identical.

6. The process according to any of claims 1 to 5, wherein all of the substituents R are phenyl.

7. The process according to any of claims 1 to 6, wherein the at least one catalyst has been selected from the group of the bases.

8. The process according to any of claims 1 to 7, wherein the base is potassium carbonate, sodium carbonate, lithium carbonate, or lithium acetate.

9. The process according to any of claims 1 to 6, wherein the at least one catalyst has been selected from the group of the Lewis acids.

10. The process according to any of claims 1 to 6, 8, or 9, wherein the Lewis acid is magnesium chloride, calcium chloride, or tetraisopropyl titanate.

11. The process according to any of claims 1 to 10, wherein the phosphate or thiophosphate of the formula (I) and the 1,4:3,6-dianhydrohexitol of the formula (III) are reacted with one another in stoichiometric or superstoichiometric amounts in a ratio of from 2:1 to 20:1, based on 1,4:3,6-dianhydrohexitol.

12. The process according to any of claims 1 to 11, which is carried out at temperatures in the range from 90 to 170°C.

13. The process according to any of claims 1 to 12, which is carried out in the absence of a diluent.

14. The process according to claim 13, which is carried out at pressures in the range from 0 to 20 mbar.

## Revendications

1. Procédé de fabrication d'un 2,5-bis(phosphate) ou -bis(thiophosphate) de 1,4:3,6-dianhydrohexitol de formule (II) 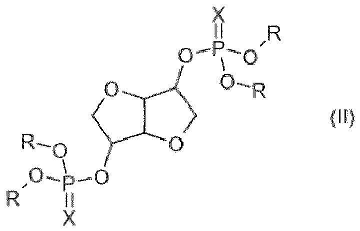 par transestérification d'au moins un phosphate ou thiophosphate de formule (I) 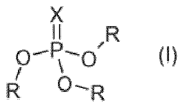 avec au moins un 1,4:3,6-dianhydrohexitol de formule (III) 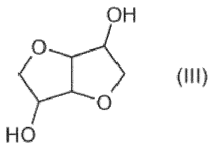 en présence d'au moins un catalyseur du groupe des bases et des acides de Lewis, les substituants X et R dans les formules (I) et (II) ayant la signification suivante :
les X sont identiques ou différents et représentent 0 ou S ;
les R sont identiques ou différents et représentent un groupe alkyle en C₁-C₁₆ linéaire ou ramifié, un groupe alcényle en C₂-C₁₆ linéaire ou ramifié, un cycloalkyle en C₃-C₁₀, un aryle en C₆-C₁₀, un benzyle, ou deux substituants R forment ensemble avec les atomes 0 auxquels ils sont reliés et l'atome P un cycle,
les substituants R étant non substitués ou substitués par un ou plusieurs radicaux OH, alkyle en C₁-C₄, CF₃, cycloalkyle en C₃-C₇, alcoxy en C₁-C₄, alcényle en C₁-C₄, aryle en C₆-C₁₀, CN.

2. Procédé selon la revendication 1, **caractérisé en ce que** tous les substituants X représentent O.

3. Procédé selon la revendication 1, **caractérisé en ce que** tous les substituants X représentent S.

4. Procédé selon l'une quelconque des revendications 1 à 3, **caractérisé en ce que** les R représentent des radicaux aryle en C₆-C₁₀ identiques ou différents.

5. Procédé selon l'une quelconque des revendications 1 à 4, **caractérisé en ce que** tous les substituants R sont identiques.

6. Procédé selon l'une quelconque des revendications 1 à 5, **caractérisé en ce que** tous les substituants R représentent phényle.

7. Procédé selon l'une quelconque des revendications 1 à 6, **caractérisé en ce que** le ou les catalyseurs sont choisis dans le groupe des bases.

8. Procédé selon l'une quelconque des revendications 1 à 7, **caractérisé en ce que** la base est le carbonate de potassium, le carbonate de sodium, le carbonate de lithium ou l'acétate de lithium.

9. Procédé selon l'une quelconque des revendications 1 à 6, **caractérisé en ce que** le ou les catalyseurs sont choisis dans le groupe des acides de Lewis.

10. Procédé selon l'une quelconque des revendications 1 à 6, 8 ou 9, **caractérisé en ce que** l'acide de Lewis est le chlorure de magnésium, le chlorure de calcium ou le titanate de tétraisopropyle.

11. Procédé selon l'une quelconque des revendications 1 à 10, **caractérisé en ce que** le phosphate ou thiophosphate de formule (I) et le 1,4:3,6-dianhydrohexitol de formule (III) sont mis en réaction l'un avec l'autre en quantités stoechiométriques ou sur-stoechiométriques en un rapport de 2:1 à 20:1, par rapport au 1,4:3,6-dianhydrohexitol.

12. Procédé selon l'une quelconque des revendications 1 à 11, **caractérisé en ce qu'**il est réalisé à des températures dans la plage allant de 90 à 170 °C.

13. Procédé selon l'une quelconque des revendications 1 à 12, **caractérisé en ce qu'**il est réalisé en l'absence d'un diluant.

14. Procédé selon la revendication 13, **caractérisé en ce qu'**il est réalisé à des pressions dans la plage allant de 0 à 20 mbar.
